(19) Europäisches Patentamt / European Patent Office / Office européen des brevets

(11) **EP 1 884 765 B1**

(12) **FASCICULE DE BREVET EUROPEEN**

(45) Date de publication et mention
de la délivrance du brevet:
**15.06.2011 Bulletin 2011/24**

(51) Int Cl.:
*G01N 21/64* (2006.01)    *A61B 5/00* (2006.01)

(21) Numéro de dépôt: **07113448.0**

(22) Date de dépôt: **30.07.2007**

(54) **Procédé et dispositif de reconstruction 3D de la distribution d'éléments fluorescents**

Verfahren und Vorrichtung zur dreidimensionalen Rekonstruktion der Verteilung von fluoreszierenden Elementen

Method and device for the 3D reconstruction of the distribution of fluorescent elements

(84) Etats contractants désignés:
**AT BE BG CH CY CZ DE DK EE ES FI FR GB GR
HU IE IS IT LI LT LU LV MC MT NL PL PT RO SE
SI SK TR**

(30) Priorité: **02.08.2006 FR 0607065**

(43) Date de publication de la demande:
**06.02.2008 Bulletin 2008/06**

(73) Titulaire: **Commissariat à l'Énergie Atomique
et aux Énergies Alternatives
75015 Paris (FR)**

(72) Inventeurs:
• **Laidevant, Aurélie
74150, RUMILLY (FR)**
• **Da Silva, Anabela
38000, GRENOBLE (FR)**
• **Dinten, Jean-Marc
69008, LYON (FR)**

(74) Mandataire: **Poulin, Gérard et al
BREVALEX
95 rue d'Amsterdam
75378 Paris Cedex 8 (FR)**

(56) Documents cités:
**WO-A-96/26431        WO-A-2005/043138
GB-A- 2 231 958       US-A1- 2002 072 677
US-B1- 6 304 771**

**Description**

**DOMAINE TECHNIQUE ET ART ANTÉRIEUR**

L'invention concerne le domaine de l'imagerie moléculaire de fluorescence sur les tissus biologiques par des méthodes optiques résolues en temps.

Elle s'applique notamment à l'imagerie moléculaire optique sur le petit animal et à l'imagerie moléculaire optique sur l'homme (cerveau, sein, autres organes où des fluorophores peuvent être injectés).

Les techniques optiques d'imagerie moléculaire de fluorescence se développent de plus en plus actuellement grâce à l'utilisation de marqueurs fluorescents spécifiques. Ceux-ci viennent se fixer de façon préférentielle sur les cellules cibles d'intérêt (par exemple des cellules cancéreuses) et offrent un meilleur contraste de détection que les marqueurs non spécifiques. Ces techniques ont pour but de localiser spatialement les marqueurs fluorescents, mais aussi d'en déterminer la concentration.

Les systèmes de tomographie optique utilisent diverses sources de lumière. Il existe donc des appareils en mode continu, des appareils en mode fréquentiel (qui utilisent des lasers modulés en fréquence) et enfin des appareils fonctionnant en mode temporel, qui utilisent des lasers pulsés.

Les données temporelles sont celles qui contiennent le plus de contenu informationnel sur le tissu traversé, mais pour lesquelles les techniques de reconstruction sont les plus complexes. La mesure en chaque point d'acquisition est en effet une fonction dépendante du temps (appelée TPSF pour « Temporal Point Spread Function », ou fonction d'étalement temporel).

On cherche à extraire des paramètres simples de la TPSF, dont on connaît par ailleurs l'expression théorique. Ensuite, la résolution du problème inverse permet de retrouver la distribution des marqueurs fluorescents.

Dans le document WO 2006/032151, on extrait les trois premiers moments des courbes temporelles, qui sont appelés les « moments normalisés » car ce sont les moments des courbes normées par la courbe d'excitation.

Or les moments des courbes temporelles sont dépendants de la connaissance du temps de vie de fluorescence. Par conséquent, dans ce document, afin de résoudre le problème inverse, le temps de vie de fluorescence est supposé connu. Or ce temps de vie peut être sensible à l'environnement et est délicat à mesurer in vivo. Un mauvais choix de ce paramètre conduit à des résultats erronés.

L'article de A. T. N. Kumar et al. "Fluorescence lifetime-based tomography for turbid media," Opt. Lett. 30(24), 3347-3349 (2005) décrit une méthode qui reconstruit un ou plusieurs temps de vie. En fait, elle est basée sur une détermination des différents temps de vie sur les courbes de déclin ; puis sur une reconstruction à partir de leurs amplitudes respectives. Une approximation sur le temps de vie est faite a priori. Cette technique est surtout intéressante si on a deux temps de vie bien distincts.

L'article "Time-Domain Fluorescence Molecular Tomography Based on Generalized Pulse Spectrum Technique", Fen GAO, Wei LIU et al., Proceedings BIOMED 2006 présente une méthode basée sur les transformées de Laplace des courbes temporelles à deux fréquences, qui nécessite deux reconstructions, une sur la concentration et l'autre sur le temps de vie. Cette méthode est plus complexe et plus coûteuse en temps de calcul car basée sur des calculs numériques pour obtenir les transformées de Laplace (on ne trouve pas facilement d'expression analytique).

Il se pose donc le problème de la détermination de la distribution spatiale de fluorophores dans un milieu diffusant, sans connaissance préalable du temps de vie de fluorescence.

Le document US 2002/72677 décrit un procédé d'imagerie de tissus ayant des propriétés de diffusion de la lumière, avec des agents de contrastes fluorescents. Il met systématiquement en oeuvre une étape de détermination de la durée de fluorescence.

Le document WO 2005/43 138 décrit un procédé et un dispositif pour déterminer la profondeur et la concentration d'un fluorophore dans un milieu trouble.

Le document US 6 304 771 décrit un dispositif et un procédé pour réaliser une imagerie de fluorophores dans un objet.

Le document WO 96/26 431 décrit une technique d'imagerie optique mettant en oeuvre une mesure de lumière diffusée.

Le document GB 2 231 958 décrit une technique de mesure de caractéristiques de fluorescence.

**EXPOSÉ DE L'INVENTION**

L'invention concerne d'abord un procédé selon la revendication 1.

Les valeurs de la différence sont indépendantes de $\tau$ mais dépendantes de la position ou de la distribution spatiale de fluorophores dans ledit milieu.

Le ou les fluorophores peuvent être fixés sur des tissus biologiques, par exemple des cellules cibles d'intérêt (par exemple des cellules cancéreuses).

La détection du signal ou des signaux est de préférence réalisée sur une fenêtre de mesure permettant de récupérer la quasi-totalité des photons émis par la fluorescence. Le calcul des valeurs d'une variable indépendante de la durée de vie peut donc se faire en tenant compte de l'intégralité du signal ou des signaux détecté(s) sur cette fenêtre de mesure.

Un procédé selon l'invention peut par exemple s'affranchir du temps de vie de fluorescence en considérant une mesure normalisée, par exemple à la mesure qui donne le plus petit temps de vie de fluorescence.

La variable indépendante de $\tau$ peut aussi résulter d'une fonction normalisée en fréquence, par exemple par rapport à un desdits signaux de fluorescence.

La détermination de la position ou de la répartition ou de la distribution spatiale de fluorophores dans le milieu peut être réalisée par une méthode d'inversion utilisant des valeurs de la différence. La méthode d'inversion peut résulter de la minimisation d'une fonction d'erreur entre la mesure et les valeurs de ladite différence, par exemple à l'aide d'une méthode de simplex.

Selon un autre mode de réalisation, la détermination de la distribution spatiale de fluorophores dans ledit milieu met en oeuvre la résolution d'un système d'équations linéaires :

$$M = P \times C,$$

où M est un vecteur colonne de mesures, P une matrice de poids et C un vecteur colonne de la répartition.

Un procédé selon l'invention peut comporter en outre une étape préalable de mesure de signal de fluorescence émis par le milieu environnant, en l'absence de fluorophore, puis une étape de correction des signaux de fluorescence ($\Phi_{fluo}$) émis par le fluorophore dans son milieu environnant.

L'invention concerne également un dispositif selon la revendication 12.

Les moyens de détection sont par exemple de type TCSPC ou des moyens de type caméra.

Un dispositif selon l'invention peut en outre comporter des moyens de représentation visuelle ou graphique de la position ou de la distribution spatiale du ou des fluorophores.

La variable indépendante de $\tau$ peut résulter d'une fonction normalisée en fréquence, par rapport à un desdits signaux de fluorescence, celui-ci pouvantêtre avantageusement celui pour lequel le temps moyen calculé est minimal.

Selon un mode de réalisation, les moyens pour déterminer la position ou la distribution spatiale de fluorophores dans le milieu mettent en oeuvre une minimisation d'une fonction d'erreur des valeurs de ladite différence, cette minimisation pouvant être un traitement par ajustement à l'aide d'une méthode de simplex.

## BRÈVE DESCRIPTION DES DESSINS

- Les figures 1A et 1B représentent chacune un exemple de dispositif expérimental pour la mise en oeuvre de l'invention,
- les figures 2A et 2B représentent respectivement une série d'impulsions laser et de photons uniques émis, et une courbe de fluorescence obtenue à partir des données relatives aux photons uniques,
- la figure 3 représente des exemples de courbes de déclins temporels de fluorescence, avec et sans fluorophore,
- la figure 4 représente schématiquement la position relative d'un couple source-détecteur et d'une inclusion de s,
- la figure 5 représente des données de fluorescence pour différentes positions d'un fluorophore sous des fibres d'excitation et de détection,
- la figure 6 représente des temps moyens calculés à partir de mesures de fluorescence pour différentes positions ou profondeurs d'un fluorophore,
- la figure 7 représente trois courbes de déclin temporel de fluorescence, l'une résultant d'une mesure, et deux de simulations,
- la figure 8 représente une grille de détecteurs,
- la figure 9 représente l'évolution de temps moyens de détection pour différentes positions de l'ensemble détecteurs-fluorophores,
- la figure 10 représente l'évolution de temps moyens de détection en fonction du chemin optique total parcouru par les photons.

## EXPOSÉ DÉTAILLÉ DE MODES DE RÉALISATION PARTICULIERS

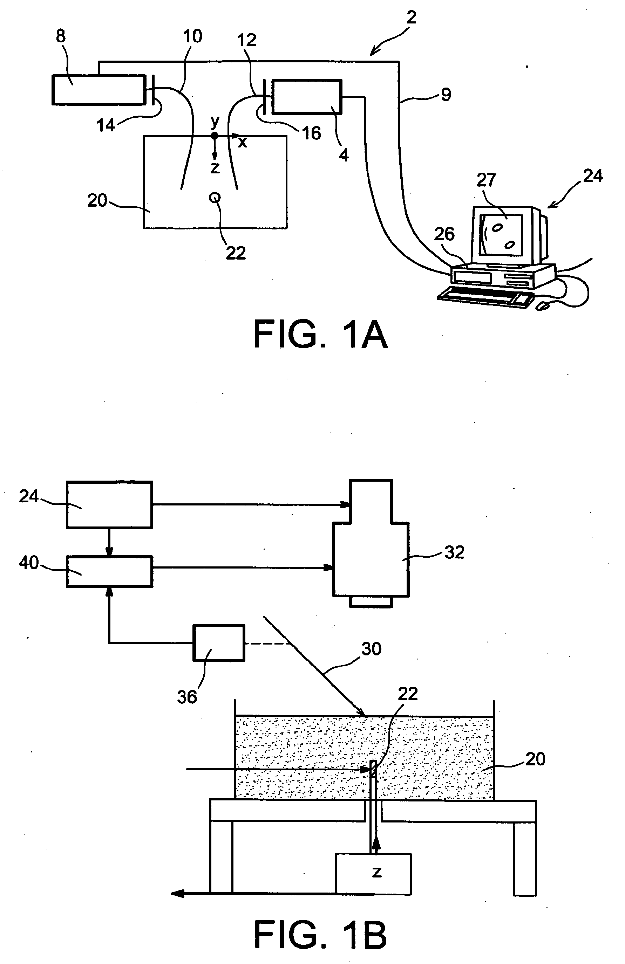

La figure 1A est un exemple de système expérimental 2 utilisant comme détecteur 4 un photomultiplicateur et une carte TCSPC (Time Correlated Single Photon Counting), en fait intégrée dans un ensemble de moyens 24 de traitement de données.

La lumière est émise par une source 8 de rayonnement en impulsions, est envoyée et collectée par des fibres 10, 12 qui peuvent être déplacées. Les deux fibres peuvent être montées sur des moyens de déplacement en translation verticaux et horizontaux (suivant les axes X et Y de la figure 1). La distance d entre les fibres est par exemple d'environ 0,2 cm.

La source 8 d'impulsions de rayonnement peut également être utilisée comme moyen de déclenchement de la carte TCSPC (voir la liaison 9 entre la source 8 et les moyens 24).

Selon un mode particulier de réalisation, la source 8 est une diode laser pulsée, à la longueur d'onde 631 nm et avec un taux de répétition de 50 MHz.

La lumière laser passe de préférence par un filtre interférentiel 14 pour éliminer toute lumière à une longueur d'onde supérieure à la longueur d'onde d'excitation.

La fibre 12 collecte la lumière en provenance du milieu 20 étudié. Un filtre interférentiel 16 et un filtre coloré absorbant les longueurs d'onde élevées peuvent être placés devant le détecteur 4 pour sélectionner la lumière de fluorescence (par exemple : $\lambda > 650$ nm, la source étant à la longueur d'onde 631 nm) d'un fluorophore 22 disposé dans le milieu 20 et optimiser l'élimination de la lumière d'excitation.

Selon la technique TCSPC (pour « Time Correlated Single Photon Counting », ou comptage de photons uniques corrélés en temps) on détecte, à l'aide du photomultiplicateur, un photon émis par le fluorophore après une impulsion de la source de rayonnement.

Le système permet donc une détection résolue en temps des impulsions de fluorescence. Il permet de récupérer la quasi-totalité des photons de fluorescence.

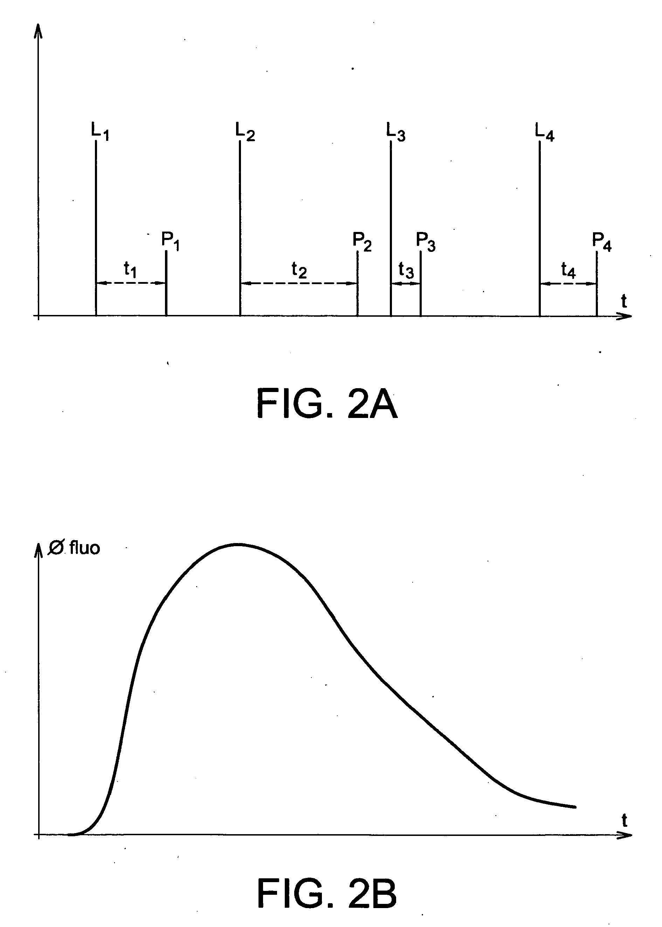

La figure 2A représente une série d'impulsions laser Li (i = 1 - 4) et une série de photons uniques pi (i = 1 - 4) correspondants. Chaque photon est en fait détecté par rapport au départ de l'impulsion correspondante : sur la figure 2A, ti représente la durée écoulée entre chaque impulsion laser Li et l'instant de détection de chaque photon pi.

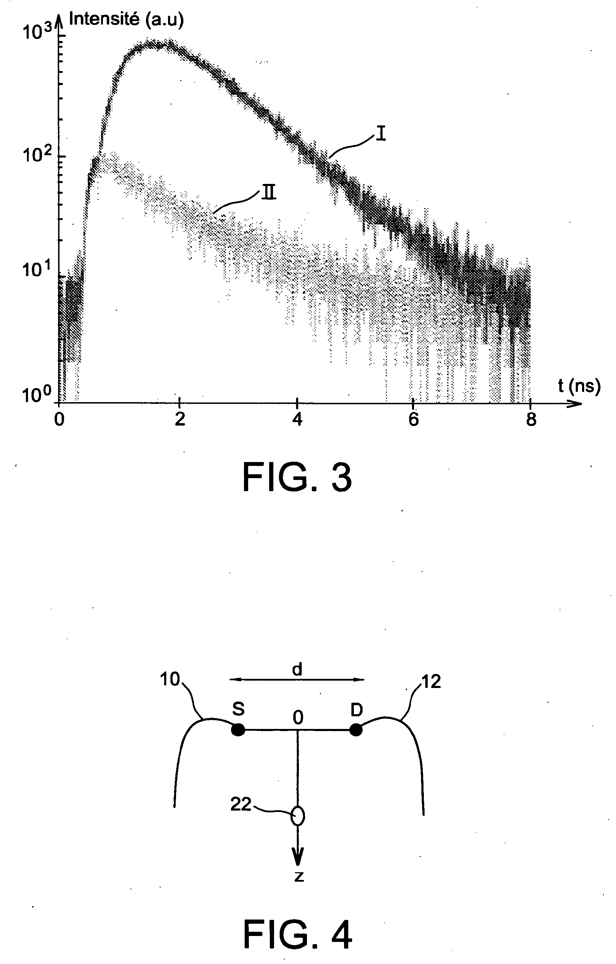

Il est donc ensuite possible d'établir une répartition statistique, comme illustré sur la figure 2B, du nombre de photons de fluorescence détectés, en fonction du temps écoulé t par rapport à chaque impulsion laser. Une telle courbe $\Phi_{fluo}$ (t) qui, on le voit (ainsi que sur les figures 3, 5 et 7), permet d'utiliser toute l'information sur une large fenêtre temporelle, de part et d'autre du point d'intensité maximum (et pas seulement dans la partie montante du signal) peut ensuite être traitée pour en tirer des informations caractéristiques comme on va l'expliquer ci-dessous.

Des moyens électroniques 24 tels qu'un micro ordinateur sont programmés pour mémoriser et traiter les données de la carte TCSPC. Plus précisément une unité centrale 26 est programmée pour mettre en oeuvre un procédé de traitement selon l'invention. Des moyens 27 d'affichage ou de visualisation permettent, après traitement, de représenter le positionnement ou la distribution spatiale des fluorophores dans le milieu 20 examiné.

D'autres techniques de détection peuvent être employées, par exemple une caméra intensifiée ultra-rapide de type « gated camera » ; dans ce cas la caméra s'ouvre sur une porte temporelle, de largeur par exemple environ 200 ps, puis cette porte est décalée, par exemple de 25 ps en 25 ps.

La figure 1B est un exemple d'un autre système expérimental 2 utilisant comme détecteur 32 une caméra rapide. Un faisceau 30 d'excitation de la fluorescence d'un milieu 20, contenant un ou plusieurs fluorophores 22, est émis par une source de rayonnement (non représentée sur la figure) qui peut être du même type que celle présentée ci-dessus en liaison avec la figure 1A. Un photodétecteur 36 permet de commander des moyens 40 formant ligne à retard. La référence 24 désigne, comme sur la figure 1A, des moyens électroniques de traitement de données de type micro-ordinateur, programmés pour mémoriser et traiter les données de la caméra 32. Une unité centrale de ces moyens 24 est programmée pour mettre en oeuvre un procédé de traitement selon l'invention. Là encore, des moyens d'affichage ou de visualisation permettent, après traitement, de représenter le positionnement ou la distribution spatiale des fluorophores dans le milieu 20 examiné.

Il est également possible de travailler avec des impulsions dans le domaine de la femto seconde, à condition de disposer de la source de rayonnement adéquate, c'est-à-dire d'une source laser 8 dont chaque impulsion a une largeur temporelle également dans le domaine de la femto seconde.

Une mesure préliminaire simple permet de mesurer la réponse (on utilise par la suite l'abréviation IRF pour « Instrument Response Function ») du dispositif : on aligne les deux fibres 10, 12, séparées par une distance prédéterminée et une impulsion est émise de la première fibre 10 vers la deuxième fibre 12. Le signal obtenu en sortie de la deuxième fibre 12 fournit la réponse IRF. La largeur à mi hauteur de ce signal, compte tenu des distances mises en jeu, peut être de l'ordre de quelques dizaines de ps, environ 80 ps dans l'exemple donné.

Un fantôme peut être utilisé, contenant de l'eau distillée dans laquelle une encre de Chine (de Dalbe, France) est introduite comme milieu absorbant et de l'«intralipide» (de Frésenius, France) comme milieu diffusant. Les concentrations relatives sont ajustées au niveau des paramètres des tissus biologiques. Le fantôme est contenu dans un cylindre en plastique de diamètre 11 cm et de hauteur 10 cm. Un volume de 1 $\mu$l de fluorophore Cy5 (de Amersham) à une concentration d'environ 10$\mu$mol.L-1 est placé à l'extrémité d'un tube capillaire fin 21 (voir figure 1B) en verre (longueur 3 cm et épaisseur 1 mm).. Le tube est inséré dans le fantôme par un trou. Les propriétés optiques du fantôme et la durée de vie du fluorophore sont mesurées par des techniques de type TCSPC (comptage de photons uniques) et sont données dans le tableau I ci-dessous.

<div align="center">Tableau I</div>

| $\mu_a$ | $\dot{\mu}_s$ | $\tau$ |
|---|---|---|
| 0.0534 cm$^{-1}$ | 11.82 cm$^{-1}$ | 1.02 ns |

Lors des mesures, les fibres sont introduites sur environ 2,5 cm dans le milieu 20, ce qui permet d'approcher la géométrie d'un milieu infini.

Des scans peuvent être réalisés à l'aide des moyens informatiques 24 (avec un logiciel tel que Labview) qui contrôlent à la fois les moyens de déplacement des fibres et la carte TCSPC (ou la caméra 32 de la figure 1B) avec un port de communication parallèle.

On prend d'abord l'exemple d'un milieu infini avec un seul fluorophore.

On considère un fluorophore ponctuel M dans le milieu, à une position (inconnue) repérée par un vecteur r dans un repère OXYZ. La source d'excitation (ou plutôt l'extrémité de la fibre 10, côté fluorophore) est localisée en $r_s$ et le détecteur (ou plutôt l'extrémité de la fibre 12, côté fluorophore) en position $r_d$. L'instant d'émission d'une impulsion est désigné par $t_0$.

La distance source-fluorophore est notée $|\mathbf{r}_s-r| = r_{sr}$ et la distance fluorophore-détecteur est notée $|\mathbf{r}-\mathbf{r}_d| = r_{rd}$.

Dans le domaine temporel, le flux de photons d'excitation à l'instant $t''$ est noté $\phi_x(r_{sr}, t''-t_0)$. L'indice $x$ correspond à la longueur d'onde d'excitation $\lambda_x$. Le fluorophore 22 absorbe la lumière d'excitation et réémet la lumière de fluorescence à l'instant $t'$, à la longueur d'onde $\lambda_m$, et avec une décroissance temporelle $\tau$. L'expression du flux de photons émis par le fluorophore localisé en $\mathbf{r}$ à l'instant $t'$ est la convolution de la fonction de propagation $\phi_x$ et de la décroissance temporelle de la fluorescence :

$$S_f(r_{sr}, t') \doteq \alpha \int_0^{t'} \phi_x(r_{sr}, t''-t_0) \frac{1}{\tau} \exp\left(-\frac{t'-t''}{\tau}\right) dt'' \qquad (1)$$

Où $\alpha$ est un facteur d'échelle qui dépend de la puissance d'illumination, de l'efficacité quantique du fluorophore.

L'équation (1) donne l'expression temporelle de l'intensité du rayonnement émis par le fluorophore. Ce rayonnement sera ensuite détecté par le détecteur 4 (ici aussi il s'agit de l'extrémité de la fibre, du côté du milieu 20), en position $\mathbf{r_d}$, à l'instant t, après avoir transité du fluorophore 22 à ce même détecteur. Le flux de photons de fluorescence résultant est proportionnel à une double convolution :

- pour la propagation de la source au fluorophore : la convolution de $\phi_x$ (à la longueur d'onde d'excitation) avec la durée de décroissance de la fluorescence, cette première convolution conduisant à l'expression S$_f$ ci-dessus (équation (1)),
- et, ensuite, celle de S$_f$ (donné par l'équation (1)) avec $\phi_m$ (à la longueur d'onde d'émission), pour la propagation du fluorophore vers le détecteur.

On peut donc écrire le flux détecté de photons de fluorescence :

$$\phi_{fluo}(r_{sr}, r_{rd}, t) = \alpha \int_0^{t} S_f(r_{sr}, t', t_0)\ \phi_m(r_{rd}, t-t')\ dt'$$
$$= \alpha \int_0^{t}\int_0^{t'} \phi_x(r_{sr}, t'-t_0) \frac{1}{\tau} \exp\left(-\frac{t'-t''}{\tau}\right) \phi_m(r_{rd}, t-t')\ dt'\,dt'' \qquad (2)$$

Pour avoir une solution simplifiée, on peut utiliser la solution pour un milieu infini, en faisant l'approximation que les coefficients optiques sont les mêmes en excitation et en émission. L'expression obtenue est la suivante :

$$\phi_{fluo}(r_{sr}, r_{rd}, t) = \alpha \int_0^t dt_e \frac{r_{sr} + r_{rd}}{r_{sr} r_{rd}} \frac{1}{[4\pi c(t - t_e)]^{3/2}} \exp[-\mu_a c(t - t_e)] \exp\left[-\frac{(r_{sr} + r_{rd})^2}{4cD(t - t_e)}\right] \frac{\exp(-t_e/\tau)}{\tau} \quad (3)$$

Où $\mu_a$ est le coefficient d'absorption, et $D$ le coefficient de diffusion. Pour plusieurs fluorophores, on utilise, comme indiqué plus loin, une intégrale sur le volume, et on fait l'hypothèse d'un milieu faiblement absorbant puisque ce modèle ne tient pas compte d'une éventuelle réabsorption et d'une éventuelle diffusion.

Cette fonction théorique $\Phi_{fluo}$ correspond à la courbe, obtenue par mesures expérimentales, de la figure 2B. Un traitement des données, par exemple un calcul de certains paramètres, peut être effectué en prenant des données sur tout un intervalle temporel, depuis le début de la partie montante de la courbe jusqu'à la fin de la partie descendante, ou au moins en prenant des valeurs dans un intervalle de part et d'autre du maximum de la courbe par exemple un intervalle temporel dont les valeurs limites sont celles correspondant sensiblement à au moins x% de l'intensité maximum de la courbe, x par exemple pouvant être égal à 1 ou 5 ou 10.. En particulier, on peut à partir de telles données calculer les moments d'ordre quelconque 0, 1,.. n (n>1).

On peut notamment extraire de cette fonction, donc de l'équation 3, la durée moyenne (ou le premier moment). Pour une fonction de distribution $g(t)$, la durée moyenne est donnée par :

$$<t> = \frac{\int_{-\infty}^{\infty} t g(t)\, dt}{\int_{-\infty}^{\infty} g(t)\, dt} \quad (4)$$

Comme on le voit, ce premier moment, de même d'ailleurs que le moment d'ordre 0, peut être calculé en tenant compte de l'intégralité, ou presque, du signal détecté, ou de la fonction de fluorescence, sur une large fenêtre de mesure, et pas seulement dans la partie croissante des courbes de fluorescence.

Pour développer cette formule, considérons, plutôt que l'expression temporelle du flux, son expression fréquentielle :

$$\Phi_{fluo}(r_{sr}, r_{rd}, \omega) = \alpha' \frac{\exp[i k (r_{sr} + r_{rd})]}{r_{sr} + r_{rd}} \frac{1}{1 - i\omega\tau} \quad (5)$$

Où $\alpha'$ est une constante indépendante de la fréquence et $k^2 = -\mu_a/D + i\omega/(cD)$. Puis, en utilisant la formule suivante :

$$<t> = i \frac{\partial \Phi}{\partial \omega}\Big|_{\omega=0} \times \frac{1}{\Phi(\omega)|_{\omega=0}} \quad (6)$$

l'expression analytique du temps moyen théorique peut être trouvée :

$$<t>_{theo} = \frac{r_{sr} + r_{rd}}{2c\sqrt{\mu_a D}} + \tau \quad (7)$$

Dans cette expression apparaît la durée de vie $\tau$ du fluorophore. La position inconnue r est contenue dans les expressions de $r_{sr}$ et $r_{rd}$.

Le traitement des données expérimentales va maintenant être décrit.

Tout d'abord, le calcul du temps moyen sur les données expérimentales peut être sensible aux perturbations dues à la lumière d'excitation ou à la fluorescence du milieu environnant le fluorophore, qui se produisent lorsque le signal de fluorescence est faible. En particulier la lumière d'excitation qui arrive en premier peut diminuer le temps moyen du signal. Afin de corriger cette perturbation, le signal peut être mesuré, mais sans fluorophore.

Cette mesure sans fluorophore est designée par $mesure_{IL}$ et est composée :

- partiellement de la lumière d'excitation qui passe par les filtres,
- et partiellement (partie longue du signal) par la fluorescence du milieu environnant, ou du fantôme sans fluorophore.

Cette mesure peut être soustraite des données mesurées avec fluorophore ($mesure_{fluo}$), si on souhaite corriger ces dernières de la lumière résiduelle, et pour obtenir le signal réel, désigné par $signal_{fluo}$ (pour indiquer la différence avec une mesure directe, voir équation 8). La soustraction permet également de supprimer le bruit de fond. En prenant en compte les expressions des durées de détection (respectivement $T_{fluo}$ pour $mesure_{fluo}$ et $T_{IL}$ pour $mesure_{IL}$), le signal de fluorescence peut être exprimé comme suit :

$$signal_{fluo} = mesure_{fluo} - \frac{T_{fluo}}{T_{IL}} mesure_{IL} \qquad (8)$$

La figure 3 donne le signal avec fluorophore (courbe I) et sans fluorophore (courbe II) pour une inclusion à une profondeur z = 0,74 cm. Les extrémités des fibres tournées vers le milieu 20 sont séparées par 0,2 cm.

Pour le calcul du premier moment les valeurs limites d'intégration sont définies par des durées définies par 1% du temps correspondant à l'amplitude maximum du TPSF, pour la limite supérieure et inférieure, de façon à s'affranchir du bruit de fond en accord avec l'étude de A. Liebert et al. « Evaluation of optical properties of highly scattering media by moments of distributions of times of flight of photons » Applied optics, vol. 42, n° 28, 5785-5792 (2003).

Plus généralement, l'invention permet de mesurer un signal sur une large fenêtre temporelle, s'étendant de part et d'autre de l'instant correspondant au maximum du signal d'intensité en fonction du temps. Des données temporelles de part et d'autre de ce maximum peuvent être utilisées pour calculer les divers moments que l'on souhaite calculer.

Pour comparer le temps moyen du signal $<t>_{signal}$ avec le temps moyen théorique [équation 7], l'IRF (fonction déjà définie ci-dessus) peut être prise en compte. Le signal est la convolution de l'IRF avec la réponse théorique ou la réponse propre du milieu diffusant. Les propriétés du premier moment montrent que le temps moyen expérimental peut être écrit comme la somme du temps moyen théorique $<t>_{theo}$ et du temps moyen IRF $<t>_{IRF}$ :

$$<t>_{signal} = <t>_{theo} + <t>_{IRF} \qquad (9)$$

Le problème inverse, c'est-à-dire la détermination de la position de l'inclusion en fonction des mesures, peut être résolu pour des mesures au-dessus de l'inclusion (voir la figure 4 qui présente la géométrie des extrémités des fibres 10, 12 et la position du fluorophore 22). La distance inter-fibres étant faible (d = 0.2 cm), on peut supposer que $r_{sr} + r_{rd} \sim 2z$, où $z$ représente la profondeur de l'inclusion sous les fibres. On peut donc réécrire l'équation 9 :

$$<t>_{signal} = \frac{r_{sr} + r_{rd}}{2c\sqrt{\mu_a D}} + \tau + <t>_{IRF} = \frac{z}{c\sqrt{\mu_a D}} + \tau + <t>_{IRF} \qquad (10)$$

Cette expression peut être inversée pour obtenir $z$ lorsque les paramètres optiques, la durée de vie et la durée moyenne de l'IRF sont connus :

$$z = c\sqrt{\mu_a D} \left( <t>_{signal} - \tau - <t>_{IRF} \right) \qquad (11)$$

Cependant cette méthode ne peut être exploitée que si les extrémités des fibres 10, 12 sont placées exactement au-dessus de l'inclusion afin de satisfaire à l'hypothèse $r_{sr} + r_{rd} \sim 2z$ et pour déterminer la durée de vie et la durée moyenne de l'IRF.

Pour éviter cette connaissance a priori de la position de l'inclusion, de la durée de vie et de la durée moyenne de l'IRF, une méthode plus générale a été développée afin de déterminer une position complètement inconnue.

Un balayage est effectué au-dessus de l'inclusion pour $N$ positions relatives fluorophore-fibres. La position relative des deux fibres, donc d'une fibre par rapport à l'autre, reste de préférence constante. La durée moyenne, $<t>_{signal,i}$, $i \in [1,N]$, est calculée pour chaque position du balayage. Puis on sélectionne et on soustrait de chaque durée moyenne une autre durée moyenne, de préférence la plus faible : $\min(<t>_{signal,i})$. Ce choix est justifié par le fait que cette durée la plus faible correspond à la position de mesure la plus proche de l'inclusion avec le meilleur rapport signal sur bruit. On considère ensuite $\Delta <t>_{signal,i}$ comme la nouvelle variable :

$$\Delta < t >_{signal, i} = < t >_{signal, i} - \min(< t >_{signal, i}), i \in [1, N])$$
$$= \frac{r_{sr, i} + r_{rd, i}}{2c\sqrt{\mu_a D}} - \frac{r_{sr, \min} + r_{rd, \min}}{2c\sqrt{\mu_a D}} \qquad (12)$$

Cette nouvelle variable est indépendante de la durée de vie et de la durée moyenne de l'IRF du fait de la différence effectuée. Elle dépend par contre de la position du fluorophore ou de la distribution spatiale des fluorophores. Dans le cas d'un seul fluorophore, cette position de l'inclusion, de coordonnées $(x,y,z)$, est ensuite définie comme celle permettant de minimiser la fonction d'erreur $\chi^2$ :

$$\chi^2(x, y, z) = \sum_i \left( \Delta < t >_{signal, i} - \Delta < t >_{theo, i} \right)^2 \qquad (13)$$

Les variables d'entrée sont les valeurs de $\Delta<t>_{signal,i}$ extraites des mesures. Elles sont comparées à la formule théorique où $\Delta<t>_{theo,i}$ est donné par :

$$\Delta < t >_{theo, i} = < t >_{theo, i} - \min(< t >_{theo, j}), i \in [1, N]) \qquad (14)$$

Où $j$ est l'indice qui repère le signal expérimental qui possède le plus petit temps moyen. La procédure d'ajustement est par exemple basée sur une méthode de simplex telle que celle décrite dans l'article de J.C.Lagarias et al. « Convergence properties of the Nelder -Mead simplex method in low dimensions », SIAM Journal on Optimization, vol. 9(1), p. 112-147, 1998.

Des résultats expérimentaux vont être présentés, qui permettent de valider le modèle ci-dessus. On présente ensuite des mesures qui montrent qu'on peut, avec la méthode selon l'invention, résoudre le problème inverse, c'est-à-dire déterminer l'emplacement de l'inclusion.

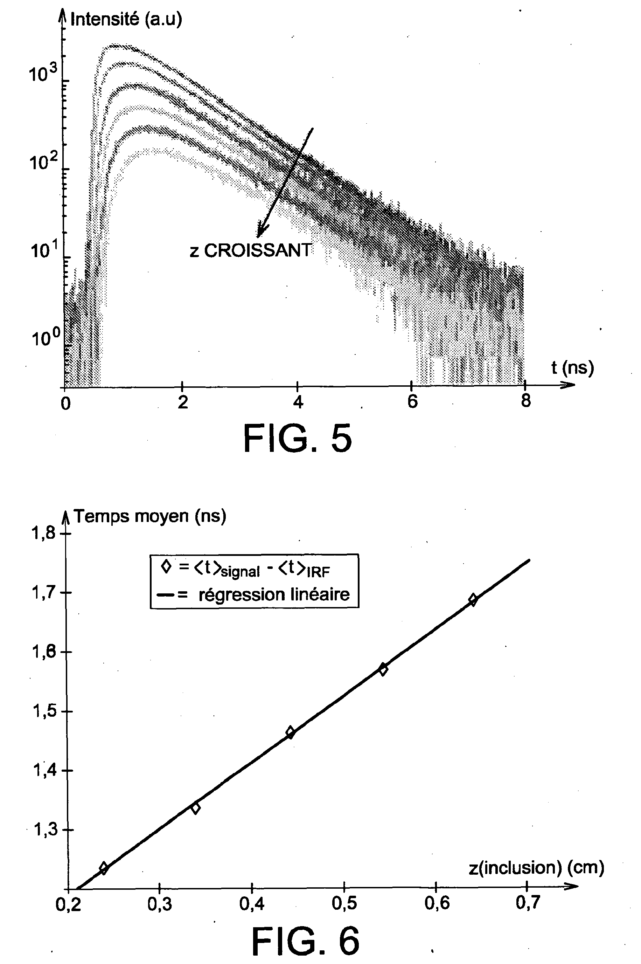

Des signaux résolus en temps pour une distance interfibre de 0,2 cm et pour des inclusions à différentes profondeurs sous les fibres ($z$= 0.24; 0.34; 0.44; 0.54; 0.64; 0.74 cm) sont présentés en échelle semi-logarithmique en figure 5.

Les amplitudes sont représentées sur cette figure en échelle arbitraire. Dans cette représentation, le déclin exponentiel de la fluorescence du Cy5 apparaît comme tendant vers une ligne droite. Plus la profondeur $z$ s'accroît, plus la position temporelle du maximum est décalée vers les durées longues, du fait du trajet plus important des photons.

L'expression théorique de la durée moyenne a d'abord été validée. La durée moyenne de l'IRF a été soustraite de la durée moyenne mesurée afin de faire une comparaison avec la formule théorique de l'équation 9.

La figure 6 représente le temps moyen expérimental corrigé de la réponse de l'instrument (= $<t>_{signal} - <t>_{IRF}$) en fonction de la profondeur $z$ de l'inclusion. En accord avec l'expression théorique (équation 10), le temps moyen est une fonction linéaire de la position de l'inclusion. La pente mesurée expérimentalement est 1.14, égale à la pente théorique $\left( c\sqrt{\mu_a D} \right)^{-1}$ calculée à partir de l'équation (10) avec les coefficients optiques du milieu.

L'ordonnée à zéro, 0.96 ns, est assez proche de la durée de vie mesurée ($\tau$=1.02 ns).

Puis, le modèle de fluorescence a été testé pour déterminer si il correspond aux mesures. La solution calculée (équation 3 avec les paramètres du tableau I) est convoluée avec l'IRF et comparée avec les mesures. Les courbes sont normalisées à la même surface que la courbe expérimentale sur un intervalle de temps arbitraire (1% du maximum sur les côtés droits et gauches).

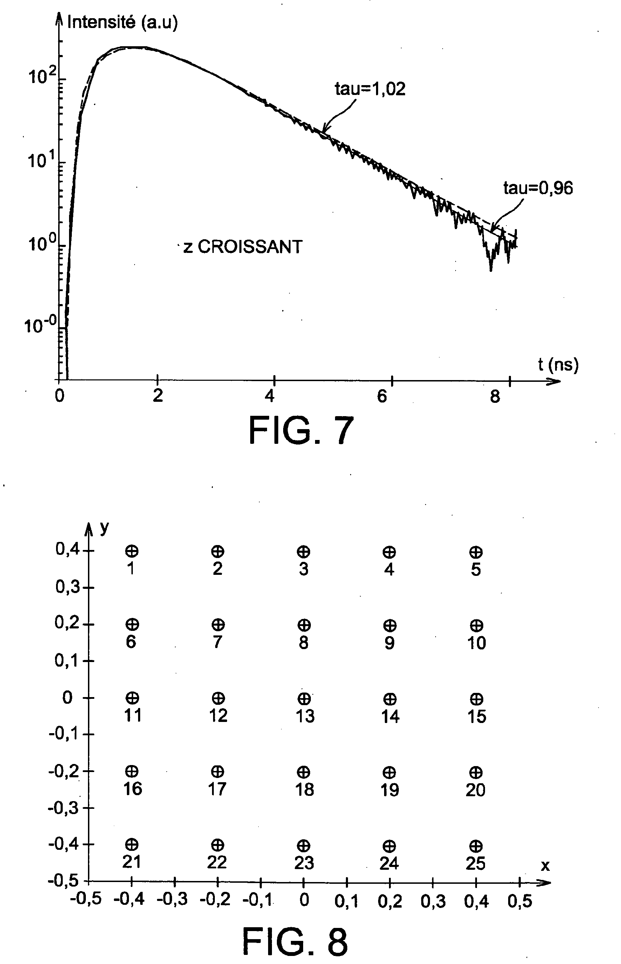

En figure 7 sont illustrés les résultats expérimentaux pour un fluorophore à la profondeur $z$ = 0.74 cm et deux simulations pour deux durées de vie différentes (0,96 ns : courbe en trait plein, 1,02 ns : courbe en traits interrompus). Un bon accord est trouvé entre les simulations et les résultats expérimentaux.

Le tableau II ci-dessous donne les résultats de la formule d'inversion pour $\tau$=0.96 ns et $\tau$=1.02 ns. On constate un bon accord entre les valeurs calculées et réelles.

Tableau II

| $z_{exp}$ (cm) | 0.24 | 0.34 | 0.44 | 0.54 | 0.64 | 0.74 |
|---|---|---|---|---|---|---|
| $z_{calculé}$($\tau$=0.96 ns) | 0.24 | 0.33 | 0.45 | 0.54 | 0.64 | 0.74 |
| $z_{calculé}$($\tau$=1.02 ns) | 0.19 | 0.28 | 0.40 | 0.49 | 0.59 | 0.69 |

La résolution du problème inverse va être illustrée pour différentes profondeurs d'inclusion en utilisant des grilles de mesure.

L'échantillonnage est effectué avec une grille de mesure comportant 5 x 5 détecteurs disposés selon un pas de 0.2 cm. La grille est positionnée au-dessus de l'inclusion. La figure 8 illustre la grille de détecteurs. La fibre 10 d'émission est à -0.2 cm (selon l'axe x) de la fibre 13 de détection. L'inclusion est centrée à (0, 0) et placée à différentes profondeurs. Le centre (0, 0) correspond à la fibre 12 de collection.

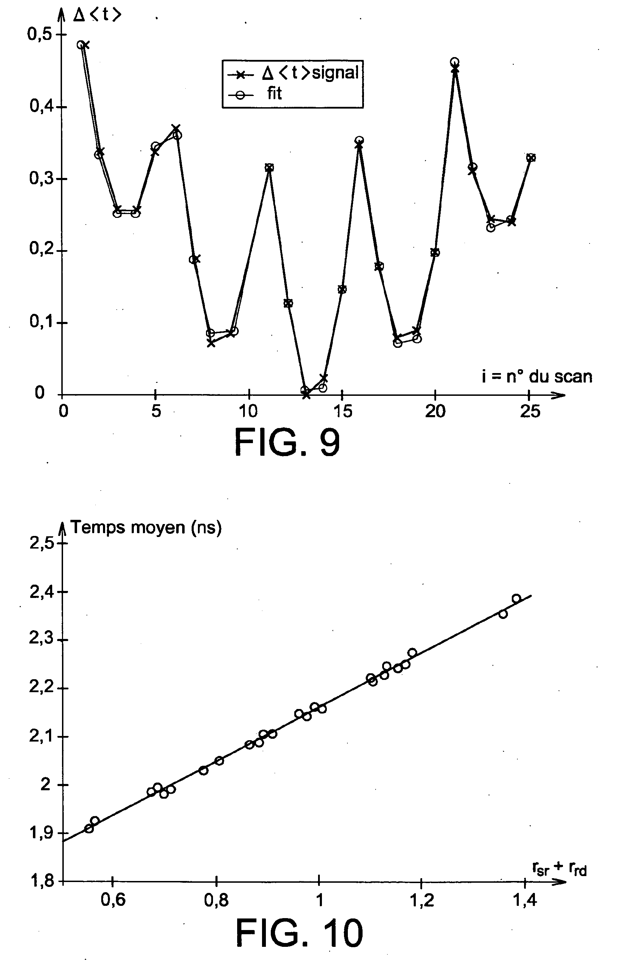

La procédure d'ajustement est appliquée aux données obtenues de tous les points de détection. Pour la première profondeur (0.24 cm), par exemple, la valeur expérimentale du temps moyen différentiel expérimental ($\Delta<t>_{signal}$) et le temps moyen différentiel calculé par ajustement sont représentés en figure 9. En figure 10, est représenté le temps moyen en fonction de $r_{sr}+r_{rd}$ (trajet des photons), pour une inclusion positionnée à z=0.24 cm. Le Tableau III montre les résultats de l'ajustement pour chaque profondeur de l'inclusion et les pentes correspondant au tracé du temps moyen en fonction de $r_{sr}+r_{rd}$. Ces pentes peuvent être comparées à la pente théorique

$$\frac{1}{2c\sqrt{\mu_a D}} \quad [\text{Eq. (7)}],$$

[Eq. (7)], calculée en utilisant les valeurs du tableau I et égale à 0.572. On retrouve donc la pente théorique, ce qui est une confirmation de la validité du modèle retenu. Le dispositif est celui de la figure 1A, mais les fibres sont séparées de 2 mm et un balayage avec un pas de 2 mm est effectué au-dessus de l'inclusion (le fantôme a un volume d'environ 1 mm$^3$). La position de l'inclusion est repérée avec une précision meilleure que le mm (200 $\mu$m sur le jeu de mesures réalisées).

Tableau III

| z réel (cm) | x (cm) | y (cm) | z (cm) | Pente |
|---|---|---|---|---|
| 0.24 | -0.01 | -0.01 | 0.24 | 0.572 |
| 0.34 | -0.02 | 0.01 | 0.33 | 0.577 |
| 0.44 | -0.02 | 0.02 | 0.42 | 0.578 |
| 0.54 | -0.02 | 0.04 | 0.53 | 0.577 |

Des scans ont été réalisés avec des grilles non centrées sur l'inclusion.

L'inclusion, positionnée initialement à z = 0,3 cm sous les fibres (position initiale = (0 ;0 ;0,34)) a été déplacée le long de l'axe x vers deux autres positions : (-0.2, 0, 0.34) puis (-0.4, 0, 0.34). La position zéro est indiquée seulement pour comparaison. Comme le montre le tableau IV, qui donne les positions obtenues par ajustement, en comparaison des positions réelles, tant la localisation *(x, y)* que la profondeur sont correctement déterminées par la procédure d'ajustement pour les trois localisations.

Tableau IV

| Position réelle | (0,0,0.3) | (-0.2, 0, 0.3) | (-0.4, 0, 0.3) |
|---|---|---|---|
| Position par ajustement | (-0.00, 0.00, 0.32) | (-0.20, 0.00, 0.32) | (-0.41, 0.00, 0.31) |

Ces essais montrent que la méthode ne nécessite pas *a priori* de connaissance de la position *(x, y)* de l'inclusion et reste valable si la grille est décalée par rapport à l'inclusion.

Expliquée ci-dessus dans le cas d'un seul fluorophore, l'invention s'applique également au cas de plusieurs fluorophores, comme va le montrer la suite. Dans ce deuxième cas, on essaie de retrouver la distribution spatiale des fluorophores dans le milieu 20. Cette distribution spatiale est la quantité à reconstruire dans le cadre du problème inverse. Le même type de dispositif expérimental que celui utilisé pour un seul fluorophore, par exemple celui de la figure 1A ou 1B, peut être utilisé dans le cas de plusieurs fluorophores.

On se place à cette fin, comme dans le premier cas, dans le cadre théorique de l'approximation de la diffusion, pour modéliser la propagation de la lumière dans le milieu diffusant. L'équation de la diffusion qui donne la densité de photons $\Phi$ pour un milieu homogène est de la forme :

$$\frac{1}{c_n}\frac{\partial\phi(r,t)}{\partial t} - D\cdot\nabla^2\phi(r,t) + \mu_a\phi(r,t) = S(r,t) \qquad (15)$$

où :

- $c_n$ est la vitesse de propagation de la lumière dans le milieu. Si on note n l'indice de réfraction du milieu, alors $c_n = c/n$ avec *c* la vitesse de propagation de la lumière dans le vide ;
- $D=1/[3(\mu_a + \mu'_s)]$ est le coefficient de diffusion, où $\mu_a$ et $\mu'_s$ sont les coefficients d'absorption et de diffusion réduits du milieu.
- S le terme de source.

Dans le domaine fréquentiel, la solution s'écrit :

$$\Phi(r,\omega) = \frac{Q_0}{4\pi D}\frac{\exp(ikr)}{r} = \frac{Q_0}{4\pi D}G(r,\omega) \qquad (16)$$

où $Q_o$ est un facteur dépendant de la puissance de la source, $G(r,\omega) = \dfrac{\exp(ikr)}{r}$ est la fonction de Green du système et où *k* est défini par la relation suivante :

$$k^2 = -\frac{\mu_a}{D} - \frac{i\omega}{cD} \qquad (17)$$

D'autres modèles peuvent aussi être utilisés (modèle par transfert radiatif, par marche aléatoire, ou simulations numériques, de type Monte Carlo par exemple). Cela signifie qu'on peut calculer une fonction de Green dans d'autres cadres que l'approximation de la diffusion.

Les équations 15 et 16 ci-dessus sont le pendant de l'expression (1) donnée ci-dessus dans le cas d'un seul fluorophore .

Considérons un milieu diffusant homogène qui contient des fluorophores.

Une impulsion source est émise en $r_s$ à $t_0$, et un détecteur est placé en $r_d$.

On note $\phi_x(|r-r_s|,t")$ la densité de photons qui atteint le point r à t". L'indice « x » indique que la longueur d'onde est celle de la source d'excitation $\lambda_x$.

Aucune hypothèse n'est nécessaire sur la forme de $\Phi_x$. On peut noter que, si le milieu est infini, $\Phi_x$ aura la forme donnée par la solution (16) en milieu infini.

Un fluorophore placé en r va absorber cette lumière $\Phi_x$ et émettre de la lumière de fluorescence à t', avec un temps de vie de fluorescence $\tau$ (aussi appelé déclin de fluorescence) et une efficacité $\eta$. La densité de photons en r à t' est la convolution de la fonction de propagation $\Phi_x$ et du déclin de fluorescence .

$$\delta S_f \left( |r - r_s|, t' \right) = \int_0^{t'} \phi_x \left( |r - r_s|, t'' - t_0 \right) \frac{\eta(r)C(r)}{\tau(r)} \exp\left( -\frac{t' - t''}{\tau(r)} \right) dt'' \qquad (18)$$

Nous l'avons noté $\delta S_f$ car ce terme devient alors un terme source à la longueur d'onde d'émission $\lambda_m$ qui va se propager et être détectée en $r_d$ à t. C(r) est proportionnel à la concentration de fluorophores au point r. D'où l'expression de la densité de photons finale $\delta\phi_{fluo}$ qui est la convolution de terme précédent et de la fonction de propagation $\Phi m$ entre l'inclusion et le détecteur :

$$\delta\phi_{fluo}\left( |r - r_s|, |r - r_d|, t \right) = \int_0^t \delta S_f \left( |r - r_s|, t' \right) \phi_m \left( |r_d - r|, t - t' \right) dt'$$

$$= \int_0^t \int_0^{t'} \phi_x \left( |r - r_s|, t'' - t_0 \right) \frac{\eta(r)C(r)}{\tau(r)} \exp\left( -\frac{t' - t''}{\tau(r)} \right) \phi_m \left( |r_d - r|, t - t' \right) dt' dt'' \qquad (19)$$

On peut adopter les notations suivantes pour alléger l'écriture :

$$r_{sr} = |r - r_s| \text{ et } , r_{rd} = |r - r_d| \qquad (20)$$

S'il y a plusieurs fluorophores dans le milieu, on intègre sur tout le volume pour obtenir l'expression finale :

$$\phi_{fluo}\left( r_{sr}, r_{rd}, t \right) = \int_v dr \delta\phi_{fl}\left( r_{sr}, r_{rd}, t \right) \qquad (21)$$

Pour cette intégration en volume on néglige les éventuelles réabsorptions et diffusions par les autres fluorophores qui ne sont pas prises en compte dans ce modèle.
Si on effectue la transformée de Fourier de l'expression précédente, on obtient l'expression de la densité de photons de fluorescence $\Phi_f(r_s, r_d, \omega)$ dans le domaine fréquentiel.
Cette expression présente l'avantage d'être un peu plus simple et bien adaptée aux calculs qui suivront :

$$\Phi_f\left( r_{sr}, r_{rd}, \omega \right) = \iiint_v \Phi_x\left( r_{sr}, \omega \right) \frac{\eta(r)C(r)}{1 + i\omega\tau(r)} \Phi_m\left( r_{rd}, \omega \right) dr^3 \qquad (22)$$

Les courbes temporelles de fluorescence sont mesurées pour un ensemble de positions relatives d'une part de l'ensemble source-détecteur et d'autre part du milieu 20. Un traitement des données, par exemple un calcul de certains paramètres, peut être effectué en prenant des données sur tout un intervalle temporel, depuis le début de la partie montante de la courbe jusqu'à la fin de la partie descendante, ou au moins en prenant des valeurs dans un intervalle de part et d'autre du maximum de la courbe, par exemple un intervalle temporel dont les valeurs limites sont celles correspondant sensiblement à au moins x% de l'intensité maximum de la courbe, x par exemple pouvant être égal à 1 ou 5 ou 10. En particulier, on peut à partir de telles données calculer les moments d'ordre quelconque 0, 1,.. n (n>1).
Si on considère un milieu infini, en chaque point, le signal mesuré est proportionnel à la densité de photons de fluorescence. La densité de photons peut être assimilée au signal en lui affectant un coefficient de proportionnalité, qui ne dépend que de facteurs instrumentaux (puissance d'excitation, gain des détecteurs, atténuation des filtres...). On exprime la densité de photons en fonction de ce facteur instrumental, des coefficients de diffusion $D$ et des fonctions de Green G qui sont connues (aux deux longueurs d'ondes qui sont toujours indiquées par les indices x et *m)* :

$$\Phi_f\left(r_{sr}, r_{rd}, \omega\right) = \alpha \iiint_V \frac{1}{D_x D_m} G_x\left(r_{sr}, \omega\right) \frac{\eta(r)C(r)}{1+i\omega\tau(r)} G_m\left(r_{rd}, \omega\right) dr^3 \quad (23)$$

Cette équation peut être discrétisée en passant à une somme sur les voxels, où $k$ représente la taille du pas de discrétisation :

$$\Phi_f\left(r_{sr}, r_{rd}, \omega\right) = \alpha \sum_{v=voxels} \frac{1}{D_x D_m} G_x\left(r_{sr_v}, \omega\right) \frac{\eta(r_v)C(r_v)}{1+i\omega\tau(r_v)} G_m\left(r_{r,d}, \omega\right) h^3 \quad (24)$$

On fait l'approximation que les coefficients optiques sont les mêmes aux deux longueurs d'onde, et on pose donc : D = Dx = Dm.

Parmi toutes les mesures, on repère le couple source-détecteur (noté $r_{s_m}$ -$r_{d_m}$) pour lequel le temps moyen de la TPSF est le plus petit : cette mesure est notée $\phi^{min}(r_{s_m}, r_{d_m})$. Elle est de préférence choisie par rapport à d'autres, car elle possède la plus grande amplitude, et donc le meilleur rapport signal sur bruit pour une puissance de la lumière d'excitation constante sur les acquisitions.

Les nouvelles fonctions considérées sont maintenant définies par:

$$\Phi^N\left(r_s, r_d, \omega\right) = \frac{\Phi_f\left(r_s, r_d, \omega\right)}{\Phi^{min}\left(r_{s_m}, r_{d_m}, \omega\right)} \quad (25)$$

Pour $\omega = 0$ la formule ci-dessus donne l'expression du moment d'ordre 0, c'est-à-dire en fait de l'intégrale dans le temps de $\Phi_{fluo}$.

Nous pouvons préciser l'expression de $\Phi^{min}(r_{s_m}, r_{d_m})$ :

$$\Phi^{min}\left(r_{s_m}, r_{d_m}, \omega\right) = \alpha \sum_{v=voxels} \frac{1}{D^2} G_x\left(r_{s_m r_v}, \omega\right) \frac{\eta(r_v)C(r_v)}{1+i\omega\tau(r_v)} G_m\left(r_{r,d}, \omega\right) h^3 \quad (26)$$

où le facteur $\alpha$ reste le même facteur que précédemment puisque les conditions expérimentales n'ont pas changé.

Rappelons ici la définition du temps moyen $m_1$ pour une distribution g(t) :

$$\dot{m}_1 = \int_{-\infty}^{\infty} t g(t) dt \Big/ \int_{-\infty}^{\infty} g(t) dt$$

Dans les notations présentées ci-dessus, le dénominateur (l'intégrale de g dans le temps) de cette expression constitue $m_0$.

Le temps moyen de la fonction $\Phi_N$ s'écrit :

$$m_1\left(\Phi^N\right) = m_1\left(\frac{\Phi_f}{\Phi^{min}}\right)$$
$$= m_1\left(\Phi_f\right) - m_1\left(\Phi^{min}\right)$$

L'expression du temps moyen $m_1(\Phi_f)$ est la suivante :

$$m_1\left(\Phi_f\right) = \frac{1}{m_0\left(\Phi_f\right)} \sum_{v=voxels} \left\{ \begin{array}{l} \left[\left(\dfrac{r_{sr_v} + r_{r,d}}{2c\sqrt{\mu_a D}} + \tau(r_v)\right)\dfrac{1}{D^2} G_x\left(r_{sr_v}, \omega = 0\right)\right] \\ \times\, G_m\left(r_{r,d}, \omega = 0\right)\alpha\, h^3 \eta\left(r_v\right) C\left(r_v\right) \end{array} \right\} \qquad (27)$$

et $m_0(\Phi_f)$ s'écrit :

$$m_0\left(\Phi_f\right) = \sum_v \frac{1}{D^2} G_x\left(r_{sr_v}, \omega = 0\right) G_m\left(r_{r,d}, \omega = 0\right)\alpha\, h^3 \eta\left(r_v\right) C\left(r_v\right) \qquad (28)$$

Cette quantité est en fait connue par la mesure : $\Phi_f$ est mesurée, et $m_0(\Phi_f)$ (ainsi d'ailleurs que $m_1(\Phi_f)$) peut être déduit de cette fonction $\Phi_f$ obtenue expérimentalement.

Le terme $\tau$ dépend de l'environnement du fluorophore. Mais on peut supposer que cet environnement est le même pour les différents fluorophores au cours d'une même mesure. Si, donc, on effectue l'hypothèse que $\tau$ est indépendant de $r_v$, ce terme $\tau$ sort de la somme et on peut simplifier l'expression de $m_1(\Phi_f)$ :

$$m_1\left(\Phi_f\right) = \frac{1}{m_0(\Phi)} \sum_v \left\{ \begin{array}{l} \left(\dfrac{r_{sr_v} + r_{r,d}}{2c\sqrt{\mu_a D}}\right)\dfrac{1}{D^2} G_x\left(r_{sr_v}, \omega = 0\right) \\ \times\, G_m\left(r_{r,d}, \omega = 0\right)\alpha\, h^3 \eta(r_v)\, C(r_v) \end{array} \right\} + \tau \qquad (29)$$

Pour $m_1(\Phi^{min})$ l'expression est la même en changeant par $\Phi$ par $\Phi_{min}$ et $r_s$ par $r_{sm}$ (respectivement rd par $r_{dm}$). Ainsi dans le calcul du temps moyen de $\Phi^N$ le temps de vie de fluorescence disparaît dans la soustraction et on obtient :

$$m_1\left(\Phi^N\right) = \frac{1}{m_0\left(\Phi_f\right)} \sum_v \left\{ \left(\frac{r_{sr_v} + r_{r,d}}{2c\sqrt{\mu_a D}}\right)\frac{1}{D^2} G_x\left(r_{sr_v}, \omega = 0\right) G_m\left(r_{r,d}, \omega = 0\right)\alpha h^3 \eta(r_v) C(r_v) \right\}$$

$$ - \frac{1}{m_0\left(\Phi^{min}\right)} \sum_v \left\{ \left(\frac{r_{s_m r_v} + r_{r,d_m}}{2c\sqrt{\mu_a D}}\right)\frac{1}{D^2} G_x\left(r_{s_m r_v}, \omega = 0\right) G_m\left(r_{r,d_m}, \omega = 0\right)\alpha h^3 \eta(r_v) C(r_v) \right\} \qquad (30)$$

Nous pouvons exprimer cette quantité de façon linéaire en fonction de $\alpha\eta(r_v)C(r_v)$, la quantité à reconstruire, en introduisant une fonction de poids :

$$m_1\left(\Phi^N\right) = \sum_v P^{mom_1}_{(r_s, r_d), r_v} \cdot \left[\alpha\eta(r_v)C(r_v)\right]$$

avec :

$$P_{(r_s,r_d),r_v}^{m_0 m_1} = \frac{1}{m_0(\Phi_f)} \left\{ \left( \frac{r_{sr_v} + r_{r_v d}}{2c\sqrt{\mu_a D}} \right) \frac{1}{D^2} G_x\big(r_{sr_v}, \omega=0\big) G_m\big(r_{r_v d}, \omega=0\big) h^3 \right\}$$

$$- \frac{1}{m_0(\Phi^{min})} \left\{ \left( \frac{r_{s_m r_v} + r_{r_v d_m}}{2c\sqrt{\mu_a D}} \right) \frac{1}{D^2} G_x\big(r_{s_m r_v}, \omega=0\big) G_m\big(r_{r_v d_m}, \omega=0\big) h^3 \right\} \quad .$$

Par conséquent, $m_1(\Phi^N)$ est mesuré, les rapports $\dfrac{1}{m_0(\Phi_f)}$ et $\dfrac{1}{m_0(\Phi^{min})}$ sont eux aussi mesurés et injectés dans la fonction de poids P qui est calculée, le système d'équations linéaires est ainsi établi, indépendamment de la valeur du temps de vie de fluorescence.

En d'autres termes, on est ramené à la résolution d'un système :

$$M = P \times C$$

Où M est un vecteur colonne de mesures, P une matrice de poids (qui dépend du modèle choisi, donc de la fonction G et des quantités $\dfrac{1}{m_0(\Phi_f)}$ et $\dfrac{1}{m_0(\Phi^{min})}$ et C un vecteur colonne des inconnues (vecteur proportionnel aux concentrations des fluorophores). Une fois le système résolu, on connaît les composantes du vecteur C qu'il est possible de représenter en 3 dimensions.

La solution peut donc être représentée à un opérateur sur des moyens de visualisation tels que les moyens 27 de la figure 1A ou 1B.

Les moyens 24 de traitement des données permettent de résoudre un système d'équations tel que le système ci-dessus, et sont donc programmés à cet effet.

La résolution du problème de la localisation ou de la distribution spatiale des fluorophores peut mettre en oeuvre une ou plusieurs des différentes techniques décrites par exemple dans A. C. Kak et al. « principles of computerized tomographic imaging », IEEE, NY, 1987.

Les données peuvent aussi être acquises en mode fréquentiel à différentes fréquences de manière à reconstruire les TPSFs en effectuant une transformée de Fourier.

Les développements ci-dessus montrent que l'on peut utiliser, dans le cadre de l'invention, une fonction de la fréquence. Cette fonction fréquentielle peut être normalisée, par exemple par rapport à la fonction fréquentielle qui correspond au temps moyen minimal.

**Revendications**

**1.** Procédé de détermination de la position d'un fluorophore (22) à durée de vie τ dans un milieu (20) environnant ou de la répartition ou de la distribution spatiale et/ou de la concentration de fluorophores (22), à durée de vie τ, dans ledit milieu (20) après excitation par un rayonnement provenant d'une source (8) de rayonnement, ou d'une source de rayonnement dont le rayonnement est envoyé par une fibre (10), ce procédé comportant :

- l'établissement, par des moyens (4) de détection et des moyens (24) de traitement de données, pour chaque couple de positions de la source (8) de rayonnement, ou d'une extrémité de la fibre (10) qui envoie le rayonnement de la source vers le milieu environnant (20), et des moyens (4, 6) de détection, ou d'une extrémité d'une fibre (12) qui amène le rayonnement de fluorescence vers les moyens de détection (8), d'un signal de fluorescence ($\Phi_{fluo}$),
- le calcul, à partir de ces signaux de fluorescence :

* des valeurs de la différence entre le temps moyen calculé pour chacun desdits signaux de fluorescence et le temps moyen calculé pour un desdits signaux de fluorescence, cette différence étant indépendante de la durée de vie $\tau$,

- la détermination de la position ou de la distribution spatiale et/ou de la concentration dudit au moins un fluorophore (22) dans ledit milieu (20) à partir des valeurs de ladite différence.

2. Procédé selon la revendication 1, comportant le calcul d'une fonction normalisée en fréquence dudit signal de fluorescence ($\Phi_{fluo}$).

3. Procédé selon la revendication 2, ladite fonction étant normalisée en fréquence par rapport à un desdits signaux de fluorescence.

4. Procédé selon l'une des revendications 1 à 3, dans lequel on calcule des valeurs de la différence entre le temps moyen calculé pour chacun desdits signaux de fluorescence et le temps moyen calculé pour le signal de fluorescence ayant un temps moyen minimal ou ayant un temps moyen calculé minimal.

5. Procédé selon l'une des revendications 1 à 4, la détermination de la position ou de la distribution spatiale de fluorophore (22) dans ledit milieu (20) étant réalisée par une méthode d'inversion utilisant des valeurs de ladite différence ou de ladite variable.

6. Procédé selon la revendication 5, comportant en outre un traitement statistique des valeurs de ladite différence de type minimisation d'une fonction d'erreur.

7. Procédé selon l'une des revendications 1 à 6, la détermination de la répartition ou de la distribution spatiale de fluorophore (22) dans ledit milieu (20) mettant en oeuvre la résolution d'un système d'équations linéaires :

$$M = P \times C,$$

où M est un vecteur colonne de mesures, P une matrice de poids et C un vecteur colonne de la répartition.

8. Procédé selon l'une des revendications 1 à 7, comportant en outre une représentation visuelle ou graphique de la position ou de la répartition du ou des fluorophores.

9. Procédé selon l'une des revendications 1 à 8, la source (8) étant de type femto-seconde.

10. Procédé selon l'une des revendications 1 à 9, les signaux de fluorescence étant détectés par technique TCSPC ou par caméra.

11. Procédé selon l'une des revendications 1 à 10, comportant en outre une étape préalable de mesure de signal de fluorescence par le milieu environnant (20), en l'absence de fluorophore (22), puis une étape de correction des signaux de fluorescence ($\Phi_{fluo}$) émis par le fluorophore dans son milieu environnant.

12. Dispositif de détermination de la position d'un fluorophore (22) à durée de vie $\tau$ dans un milieu (20) environnant ou de la répartition ou de la distribution spatiale et/ou de la concentration de fluorophores (22), à durée de vie $\tau$, dans ledit milieu (20) comportant :

- une source (8) de rayonnement pour l'excitation dudit fluorophore, cette source étant éventuellement munie d'une fibre (10) qui permet d'envoyer un rayonnement de la source vers le milieu environnant,
- des moyens (4) de détection, muni éventuellement d'une fibre (12) pour amener un rayonnement de fluorescence vers les moyens de détection (8), pour détecter un signal de fluorescence émis par ledit fluorophore,
- des moyens pour effectuer un déplacement relatif :

* de la source (8), ou d'une extrémité de la fibre (10) qui permet d'envoyer un rayonnement de la source vers le milieu environnant,
* et des moyens (4) de détection par rapport au fluorophore, ou d'une extrémité de la fibre (12) qui amène le rayonnement de fluorescence vers les moyens de détection (4),

- des moyens pour calculer, à partir d'une pluralité de signaux de fluorescence ($\Phi_{fluo}$),

  * des valeurs de la différence entre le temps moyen calculé pour chacun desdits signaux de fluorescence et le temps moyen calculé pour un desdits signaux de fluorescence, cette différence étant indépendante de $\tau$,

chaque signal étant établi pour une position relative :

  * d'une part du fluorophore,
  * et, d'autre part,

    ◦ de la source (8) ou d'une extrémité de la fibre (10) qui permet d'envoyer un rayonnement de la source vers le milieu environnant,
    ◦ et des moyens (4) de détection, ou d'une extrémité de la fibre (12) qui amène le rayonnement de fluorescence vers les moyens de détection (4) ;

- des moyens pour déterminer la position ou la répartition ou la distribution spatiale et/ou la concentration de fluorophore (22) dans ledit milieu (20) à partir des valeurs de ladite différence.

**13.** Dispositif selon la revendication 12, lesdits moyens de détection comportant des moyens de type TCSPC ou des moyens de type caméra.

**14.** Dispositif selon l'une des revendications 12 ou 13, comportant en outre des moyens (27) de représentation visuelle ou graphique de la position ou de la distribution spatiale du ou des fluorophores.

**15.** Dispositif selon l'une des revendications 12 à 14, comportant des moyens de calcul d'une fonction normalisée en fréquence desdits signaux de fluorescence ($\Phi_{fluo}$).

**16.** Dispositif selon la revendication 15, ladite fonction étant normalisée en fréquence par apport à un desdits signaux de fluorescence.

**17.** Dispositif selon l'une des revendications 12 à 16, dans lequel sont calculées des valeurs de la différence entre le temps moyen calculé pour chacun desdits signaux de fluorescence et le temps moyen calculé pour le signal de fluorescence ayant un temps moyen minimal ou un temps moyen calculé minimal.

**18.** Dispositif selon l'une des revendications 12 à 17, lesdits moyens pour déterminer la position ou la distribution spatiale de fluorophore (22) dans ledit milieu (20) mettant en oeuvre une méthode d'inversion utilisant des valeurs de ladite différence.

**19.** Dispositif selon la revendication précédente, lesdits moyens pour déterminer la position ou la distribution spatiale de fluorophore (22) dans ledit milieu (20) mettant en oeuvre un traitement statistique des valeurs de ladite différence comportant une minimisation d'une fonction d'erreur.

**Claims**

**1.** Method for determining the position or the spatial distribution or repartition and/or the concentration of a fluorophore with a lifetime $\tau$ in a surrounding medium (20), after excitation by radiation from a radiation source (8), or from a radiation source (8) the radiation of which is sent through a fibre, which method comprising:

- establishing, with detection means (4) and data processing means (24), for each pair of positions of the radiation source (8), or of the end of a fibre which sends said radiation from the source to the surrounding medium (20), and of the detection means (4, 6), or of the end of a fibre (12) which transmits fluorescent light towards said detection means (8), of a fluorescence signal ($\Phi_{fluo}$),
- the calculation, based on these fluorescence signals, of values of the difference between the mean time calculated for each of said fluorescence signal and the mean time calculated for one of said fluorescence signal,

EP 1 884 765 B1

said difference being independent of the lifetime $\tau$,
- the determination of the position or the spatial distribution and/or the concentration of said at least one fluorophore (22) in said medium (20) on the basis of the values of said difference.

2. Method according to claim 1, comprising the calculation of a frequency normalised function of said fluorescence signal ($\Phi_{fluo}$).

3. Method according to claim 2, said function being frequency normalised with respect to one of said fluorescence signals.

4. Method according to any of claims 1 to 3, in which values of the difference between the mean time calculated for each of said fluorescence signals and the mean time calculated for the fluorescence signal having a minimum mean time or having a minimum calculated mean time are calculated.

5. Method according to any of claims 1 to 4, the determination of the position or the spatial distribution of fluorophore (22) in said medium (20) being achieved by a method of reversal using values of said difference or said variable.

6. Method according to claim 5, further comprising a statistical processing of the values of said difference of the type of the minimisation of an error function.

7. Method according to any of claims 1 to 6, the determination of the spatial repartition or distribution of fluorophore (22) in said medium, implementing the resolution of a system of linear equations:

$$M = P \times C,$$

where M is a measurement column vector, P is a weight matrix and C is a distribution column vector.

8. Method according to any of claims 1 to 7, further comprising a visual or graphic representation of the position or the distribution of the fluorophore(s).

9. Method according to any of claims 1 to 8, the source (8) being of the femtosecond type.

10. Method according to any of claims 1 to 9, the fluorescence signals being detected by a TCSPC technique or by camera.

11. Method according to any of claims 1 to 10, further comprising a preliminary step of measuring the fluorescence signal by the surrounding medium (20), in the absence of fluorophore (22), then a step of correcting the fluorescence signals ($\Phi_{fluo}$) emitted by the fluorophore in its surrounding medium.

12. Device for determining the position or the spatial distribution or répartition and/or the concentration of a fluorophore (22) with a lifetime $\tau$ in a surrounding medium (20), comprising:

- a source (8) of radiation for excitation of said fluorophore, possibly equipped with a fibre (10) for sending said radiation from the source to the surrounding medium,
- detection means (4), possibly equipped with a fibre (12) for sending a fluorescence radiation to said detection means (4), to detect a fluorescence signal emitted by said fluorophore;
- means for performing a relative movement:

* of the source (8), or of an end of the fibre (10) for sending said radiation from the source to the surrounding medium,
* and of said detection means (4), with respect to the flurophore or of an end of the fibre (12) for sending a fluorescence radiation to said detection means (4),

- means for calculating, on the basis of a plurality of fluorescence signals $\Phi_{fluo}$, values of the difference between the mean time calculated for each of said fluorescence signal and the mean time calculated for one of said fluorescence signal, said difference being independent of the lifetime $\tau$, each signal being established for a

17

relative position:
- On the one hand of the fluorophore;
- and, on the other hand:

˚of the source (8) or of an end of the fibre (10) for sending a radiation from the source to the surrounding medium,

* and of said detection means (4), or of an end of the fibre (12) for sending a fluorescence radiation to said detection means (4);

- means for determining the position or the spatial distribution and/or the concentration of fluorophore (22) in said medium (20) on the basis of the values of said difference.

13. Device according to claim 12, said detection means comprising TCSPC-type means or camera-type means.

14. Device according to claim 12 or 13, further comprising means (27) for visual or graphic representation of the position or the spatial distribution of the fluorophore(s).

15. Device according to any of claims 12 to 14, comprising means for calculating a frequency normalized function of said fluorescence signals.

16. Device according to claim 15, said function being frequency normalised with respect to one of said fluorescence signals.

17. Device according to any of claims 12 to 16, in which values of the difference between the mean time calculated for each of said fluorescence signals and the mean time calculated for the fluorescence signal having a minimum mean time or having a minimum calculated mean time are claculated.

18. Device according to any of claims 12 to 17, said means for determining the position or the spatial distribution of fluorophore (22) in said medium (20) implementing a method of reversal using values of said difference.

19. Device according to the preceding claim, said means for determining the position or the spatial distribution of fluorophore (22) in said medium (20) implementing a statistical processing of the values of said difference comprising minimisation of an error function.

**Patentansprüche**

1. Verfahren zur Bestimmung der Position eines Fluorophors (22) mit Lebensdauer $\tau$ in einem Umgebungsmedium (20) oder der Verteilung oder der räumlichen Verteilung oder/und der Konzentration von Fluorophoren (22) mit Lebensdauer $\tau$ in dem Medium (20) nach Anregung durch eine Strahlung, welche von einer Strahlungsquelle (8) ausgeht oder von einer Strahlungsquelle, deren Strahlung durch eine Faser (10) geführt wird, wobei das Verfahren umfasst:

- Bilden eines Fluoreszenz-Signals ($\Phi_{fluo}$) durch Mittel (4) zur Detektion und Mittel (24) zur Verarbeitung von Daten für jedes Paar von Positionen von der Strahlungsquelle (8) oder von einem Endabschnitt der Faser (10), die die Strahlung von der Quelle zu dem Umgebungsmedium (20) hin führt, und Mittel (4, 6) zur Detektion oder von einem Endabschnitt einer Faser (12), die die Fluoreszenz-Strahlung zu den Detektionsmitteln (8) hin führt,
- ausgehend von diesen Fluoreszenz-Signalen Berechnen von Werten der Differenz zwischen der für jedes der Fluoreszenz-Signale berechneten mittleren Zeit und der für eines der Fluoreszenz-Signale berechneten mittleren Zeit, wobei diese Differenz unabhängig von der Lebensdauer $\tau$ ist,
- Bestimmen der Position oder der räumlichen Verteilung oder/und der Konzentration des wenigstens einen Fluorophors (22) in dem Medium (20) ausgehend von den Werten der Differenz.

2. Verfahren nach Anspruch 1, umfassend das Berechnen einer in Bezug auf das Fluoreszenz-Signal ($\Phi_{fluo}$) frequenz-normierten Funktion.

3. Verfahren nach Anspruch 2, wobei die Funktion in Bezug auf eines der Fluoreszenz-Signale frequenznormiert wird.

4. Verfahren nach einem der Ansprüche 1 bis 3, wobei Werte der Differenz zwischen der für jedes der Fluoreszenz-Signale berechneten mittleren Zeit und der mittleren Zeit, welche für das Fluoreszenz-Signal berechnet wird, das eine minimale mittlere Zeit oder eine minimale berechnete mittlere Zeit aufweist, berechnet werden.

5. Verfahren nach einem der Ansprüche 1 bis 4, wobei die Bestimmung der Position oder der räumlichen Verteilung von Fluorophor (22) in dem Medium (20) durch ein Inversionsverfahren unter Verwendung von Werten der Differenz oder der Variable durchgeführt wird.

6. Verfahren nach Anspruch 5, ferner umfassend eine statistische Verarbeitung der Werte der Differenz von der Art einer Minimierung einer Fehlerfunktion.

7. Verfahren nach einem der Ansprüche 1 bis 6, wobei die Bestimmung der Verteilung oder der räumlichen Verteilung von Fluorophor (22) in dem Medium (20) die Lösung eines linearen Gleichungssystems M = P x C ausführt, wobei M ein Mess-Spaltenvektor, P eine Gewichtungsmatrix und C ein Verteilungs-Spaltenvektor ist.

8. Verfahren nach einem der Ansprüche 1 bis 7, ferner umfassend eine visuelle oder graphische Darstellung der Position oder der Verteilung des Fluorophors oder der Fluorophore.

9. Verfahren nach einem der Ansprüche 1 bis 8, wobei die Quelle (8) von Femtosekunden-Art ist.

10. Verfahren nach einem der Ansprüche 1 bis 9, wobei die Fluoreszenz-Signale durch eine TCSPC-Technik oder durch eine Kamera detektiert werden.

11. Verfahren nach einem der Ansprüche 1 bis 10, ferner umfassend einen vorherigen Schritt einer Messung von Fluoreszenz-Signalen von dem Umgebungsmedium (20) in Abwesenheit von Fluorophor (22), dann einen Schritt einer Korrektur der von dem Fluorophor in seinem Umgebungsmedium emittierten Fluoreszenz-Signale ($\Phi_{fluo}$).

12. Vorrichtung zur Bestimmung der Position eines Fluorophors (22) mit Lebensdauer $\tau$ in einem Umgebungsmedium (20) oder der Verteilung oder der räumlichen Verteilung oder/und der Konzentration von Fluorophoren (22) mit Lebensdauer $\tau$ in dem Medium (20), umfassend:

- eine Strahlungsquelle (8) zur Anregung des Fluorophors, wobei die Quelle möglicherweise mit einer Faser (10) bereitgestellt ist, die es ermöglicht, eine Strahlung von der Quelle zu dem Umgebungsmedium hin zu führen,
- Detektionsmittel (4), die möglicherweise mit einer Faser (12) zur Leitung einer Fluoreszenz-Strahlung zu den Detektionsmitteln (8) hin bereitgestellt sind, um ein von dem Fluorophor emittiertes Fluoreszenz-Signal zu detektieren,
- Mittel zum Bewirken einer Relativverlagerung

* von der Quelle (8) oder von einem Endabschnitt der Faser (10), die es ermöglicht, eine Strahlung von der Quelle zu dem Umgebungsmedium hin zu führen, und
* Mittel (4) zur Detektion in Bezug auf den Fluorophor oder von einem Endabschnitt der Faser (12), die die Fluoreszenz-Strahlung zu den Detektionsmitteln (4) hin führt,

- ausgehend von einer Mehrzahl von Fluoreszenz-Signalen ($\Phi_{fluo}$) Mittel zum Berechnen von:

* Werten der Differenz zwischen der für jedes der Fluoreszenz-Signale berechneten mittleren Zeit und der für eines der Fluoreszenz-Signale berechneten mittleren Zeit, wobei diese Differenz unabhängig von $\tau$ ist, wobei jedes Signal für eine Relativposition gebildet ist von:
* dem Fluorophor einerseits,
* und andererseits

- von der Quelle (8) oder von einem Endabschnitt der Faser (10), die es ermöglicht, eine Strahlung von der Quelle zu dem Umgebungsmedium hin zu führen,
- und von den Detektionsmitteln (4) oder von einem Endabschnitt der Faser (12), die die Fluoreszenz-Strahlung zu den Detektionsmitteln (4) hin führt,

- Mittel zum Bestimmen der Position oder der Verteilung oder der räumlichen Verteilung oder/und der Konzentration des Fluorophors (22) in dem Medium (20) ausgehend von den Werten der Differenz.

**13.** Vorrichtung nach Anspruch 12, wobei die Detektionsmittel Mittel von einer TCSPC-Art oder Mittel von der Art einer Kamera umfassen.

**14.** Vorrichtung nach einem der Ansprüche 12 oder 13, ferner umfassend Mittel (27) zur visuellen oder graphischen Darstellung der Position oder der räumlichen Verteilung des Fluorophors oder der Fluorophore.

**15.** Vorrichtung nach einem der Ansprüche 12 bis 14, umfassend Mittel zur Berechnung einer in Bezug auf die Fluoreszenz-Signale ($\Phi_{fluo}$) frequenznormierten Funktion.

**16.** Vorrichtung nach Anspruch 15, wobei die Funktion frequenznormiert in Bezug auf eines der Fluoreszenz-Signale ist.

**17.** Vorrichtung nach einem der Ansprüche 12 bis 16, wobei Werte der Differenz zwischen der für jedes der Fluoreszenz-Signale berechneten mittleren Zeit und der für das Fluoreszenz-Signal mit einer minimalen mittleren Zeit oder einer minimalen berechneten mittleren Zeit berechneten mittleren Zeit berechnet werden.

**18.** Vorrichtung nach einem der Ansprüche 12 bis 17, wobei die Mittel zur Bestimmung der Position oder der räumlichen Verteilung von Fluorophor (22) in dem Medium (20) ein Inversionsverfahren unter Verwendung von Werten der Differenz ausführen.

**19.** Verfahren nach dem vorhergehenden Anspruch, wobei die Mittel zur Bestimmung der Position oder der räumlichen Verteilung des Fluorophors (22) in dem Medium (20) eine statistische Verarbeitung der Werte der Differenz ausführen, welche eine Minimierung einer Fehlerfunktion umfasst.

FIG. 1A

FIG. 1B

FIG. 2A

FIG. 2B

FIG. 3

FIG. 4

FIG. 5

FIG. 6

FIG. 7

FIG. 8

FIG. 9

FIG. 10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006032151 A
- US 200272677 B
- WO 200543138 A
- US 6304771 B
- WO 9626431 A
- GB 2231958 A

**Littérature non-brevet citée dans la description**

- **A. T. N. KUMAR et al.** Fluorescence lifetime-based tomography for turbid media. *Opt. Lett.,* 2005, vol. 30 (24), 3347-3349
- **Fen GAO ; Wei LIU et al.** Time-Domain Fluorescence Molecular Tomography Based on Generalized Pulse Spectrum Technique. *Proceedings BIOMED,* 2006
- **A. LIEBERT et al.** Evaluation of optical properties of highly scattering media by moments of distributions of times of flight of photons. *Applied optics,* 2003, vol. 42 (28), 5785-5792
- **J.C.LAGARIAS et al.** Convergence properties of the Nelder -Mead simplex method in low dimensions. *SIAM Journal on Optimization,* 1998, vol. 9 (1), 112-147
- **A. C. KAK et al.** principles of computerized tomographic imaging. IEEE, 1987